# EUROPÄISCHE PATENTANMELDUNG

(11) **EP 4 059 595 A1**
(43) Veröffentlichungstag der Anmeldung: **21.09.2022**
(21) Anmeldenummer: 21162659.3
(22) Anmeldetag: 15.03.2021
(51) Int. Cl.: B01J 8/06, B01J 19/30, C07C 11/04, C07C 5/333

(54) **HERSTELLUNG VON ETHYLEN DURCH OXIDATIVE DEHYDRIERUNG VON ETHAN**

(71) Anmelder: Linde GmbH, 82049 Pullach (DE)
(72) Erfinder: Zellhuber, Mathieu, 82152 Martinsried (DE); Schubert, Martin, 81375 München (DE); Meiswinkel, Andreas, 83253 Rimsting (DE)
(74) Vertreter: DehnsGermany Partnerschaft von Patentanwälten

(57) **Zusammenfassung**

Ein Verfahren zur Herstellung von Ethylen durch oxidative Dehydrierung von Ethan, bei dem ein Rohrbündelreaktor (10) mit Reaktionsrohren (11) verwendet wird, die sich zwischen einem ersten Ende (11A) und einem zweiten Ende (11B) erstrecken, wird vorgeschlagen, wobei in jedem der Reaktionsrohre (11) ein oder mehrere Katalysatorbetten angeordnet sind und in jedem der Reaktionsrohre (11) ein Verhältnis einer Gesamtlänge des einen oder der mehreren Katalysatorbetten zwischen dem ersten Ende (11A) und dem zweiten Ende (11B) zu einem Durchmesser jedes der Reaktionsrohre (11) einen Wert zwischen 150 und 400 aufweist. Der Rohrbündelreaktor (10) wird mit einer Lineargeschwindigkeit von 250 bis 800 cm/s betrieben und das eine oder die mehreren Katalysatorbetten werden derart ausgestaltet, dass ein Verhältnis von aktiver Katalysatormasse zu wirksamer Kühlfläche in einem Bereich zwischen 1,5 und 5 kg/m² liegt. Eine entsprechende Anlage (100) ist ebenfalls Gegenstand der Erfindung.

## Beschreibung

Die vorliegende Erfindung betrifft ein Verfahren und eine Anlage zur Herstellung von Ethylen durch oxidative Dehydrierung von Ethan.

### Hintergrund der Erfindung

Die oxidative Dehydrierung (engl. Oxidative Dehydrogenation, ODH) von Paraffinen mit zwei bis vier Kohlenstoffatomen ist grundsätzlich bekannt. Bei der ODH werden die genannten Paraffine mit Sauerstoff unter anderem zu den jeweiligen Olefinen und zu Wasser umgesetzt. Die vorliegende Erfindung betrifft die oxidative Dehydrierung von Ethan zu Ethylen, nachfolgend auch als ODHE bezeichnet.

Die ODH kann gegenüber etablierteren Verfahren zur Herstellung von Olefinen wie dem Steamcracken oder der katalytischen Dehydrierung vorteilhaft sein. So besteht aufgrund der Exothermie der beteiligten Reaktionen und der praktisch irreversiblen Wasserbildung keine thermodynamische Gleichgewichtslimitierung. Die ODH kann bei vergleichsweise geringen Reaktionstemperaturen durchgeführt werden. Grundsätzlich ist keine Regenerierung der eingesetzten Katalysatoren erforderlich, da die Anwesenheit von Sauerstoff eine in-situ-Regenerierung ermöglicht bzw. bewirkt. Schließlich werden im Gegensatz zum Steamcracken geringere Mengen an wertlosen Nebenprodukten wie Koks gebildet.

Zu weiteren Details bezüglich der ODH sei auf einschlägige Fachliteratur, beispielsweise Ivars, F. und López Nieto, J. M., Light Alkanes Oxidation: Targets Reached and Current Challenges, in: Duprez, D. und Cavani, F. (Hrsg.), Handbook of Advanced Methods and Processes in Oxidation Catalysis: From Laboratory to Industry, London 2014: Imperial College Press, Seiten 767-834, oder Gärtner, C.A. et al., Oxidative Dehydrogenation of Ethane: Common Principles and Mechanistic Aspects, ChemCatChem, Bd. 5, Nr. 11, 2013, Seiten 3196 bis 3217, sowie X. Li, E. Iglesia, Kinetics and Mechanism of Ethane Oxidation to Acetic Acid on Catalysts Based on Mo-V-Nb Oxides, J. Phys. Chem. C, 2008, 112, 15001-15008, verwiesen.

Bei der ODH werden insbesondere bei Verwendung von MoVNbOₓ- und MoVNbTeOₓ - basierten Katalysatoren unter industriell relevanten Reaktionsbedingungen signifikante Mengen der jeweiligen Carbonsäuren der eingesetzten Paraffine, im Falle der ODHE insbesondere Essigsäure, als Nebenprodukte gebildet. Für einen wirtschaftlichen Anlagenbetrieb ist daher eine Koppelproduktion von Olefinen und der Carbonsäuren bei Verwendung des beschriebenen Katalysatortyps in der Regel unvermeidlich.

Durch die Bildung von Essigsäure ist die Anwendungsmöglichkeit der ODHE durch die nicht immer gegebenen Verwertungsmöglichkeiten dieses Koppelprodukts begrenzt, wohingegen für Ethylen oft deutlich größere Mengen wünschenswert wären. Nur in Einzelfällen ist eine direkte Verknüpfung der ODHE mit Prozessen, die Ethylen und Essigsäure zugleich benötigen, möglich, z.B. bei der Vinylacetat-Monomer-(VAM-) Produktion, wie sie u.a. in der WO 2018/114747 A1 und der WO 2018/114752 A1 beschrieben ist. Auch solche Ansätze beinhalten jedoch nur eine Verwertung der Essigsäure und nicht eine Lösung des Problems der Koppelproduktion.

Die WO2018/115416A1 beschreibt zwar bereits eine Möglichkeit, das Produktverhältnis zwischen Ethylen und Essigsäure bei der ODHE innerhalb gewisser Grenzen durch Anpassung des Wasserpartialdrucks im Produktgas bedarfsgerecht anzupassen, jedoch entsteht auch bei einer entsprechenden Anpassung weiterhin eine signifikante Menge an Essigsäure.

In der WO 2018/114900 A1 sind Reaktorgeometrien für einen Rohrreaktor beschrieben, wobei insbesondere Rohrgeometrien mit einer Länge von 4 bis 12 m und einem Durchmesser von 15 bis 25 mm beansprucht werden. Auch unterschiedliche Katalysatorgeometrien sind offenbart. Es werden MoVNbOₓ-basierte Katalysatoren verwendet und auch MoVNbTeOₓ -Katalysatoren sind explizit als Option erwähnt. Es findet sich jedoch keinerlei Hinweis auf die Auswirkung von Parameteränderungen wie beispielsweise erhöhter Lineargeschwindigkeiten auf die Produktverteilung. Hintergrund der WO 2018/114900 A1 ist ein Reaktorsystem, das eine ausreichend isotherme Reaktionsführung bei minimiertem Druckverlust ermöglicht. Vor diesem Hintergrund führt diese Schrift aus, dass besonders hohe Lineargeschwindigkeiten eher nachteilig seien, da diese einen erhöhten Druckverlust bedingen, der sich insbesondere bei Niedrigdruckprozessen wie der ODHE nachteilig auswirkt. Daher wird in einer weiteren Schrift, der US 9,963,412 B2 bzw. WO 2015/082598 A1, die Lineargeschwindigkeit anspruchsgemäß auf 500 cm/s nach oben begrenzt.

Die WO 2020/074750 A1 beinhaltet Angaben zu Pilotversuchen in einem Einzelrohrreaktor im industriellen Maßstab mit einem Durchmesser von 19 mm und einer Länge von 5,6 m. Die in dieser Schrift offenbarten Gegenstände beziehen sich auf die Zusammensetzung der Inertanteils im Prozessgas. Es werden keine Angaben zur Lineargeschwindigkeit gemacht, des Weiteren werden in allen drei vorgenommenen Versuchen die Bedingungen bis auf die Inertzusammensetzung und die Reaktortemperatur kaum variiert.

Zusammengefasst kann festgestellt werden, dass bekannte Optimierungen des Katalysators und bisherige Prozessoptimierungen nur bedingt zu einer Verbesserung der Ethylenausbeute führen. Es sind also weitere Maßnahmen wünschenswert, die über den Stand der Technik hinausgehen, der bislang nur eine bedarfsgerechte Anpassung der Koppelprodukte innerhalb gewisser Grenzen bzw. die Nutzung beider Produkte in Folgeprozessen ermöglicht.

Die vorliegende Erfindung stellt sich also die Aufgabe, ein Reaktorsetup und Betriebsbedingungen bereitzustellen, die es ermöglichen, unter großtechnischen Bedingungen eine maximale ODH-E-Prozessintensivierung, insbesondere mit Fokus auf optimierter Ethylenausbeute, zu erreichen.

### Offenbarung der Erfindung

Die vorstehend genannte Aufgabe wird durch ein Verfahren und eine Anlage mit den Merkmalen der jeweiligen unabhängigen Patentansprüche gelöst. Bevorzugte Ausgestaltungen der Erfindung sind jeweils Gegenstand der abhängigen Patentansprüche und der nachfolgenden Beschreibung.

Vor der Erläuterung der Merkmale und Vorteile der vorliegenden Erfindung werden zunächst noch weitere Grundlagen der Erfindung erläutert und bei der Beschreibung der Erfindung verwendete Begriffe eingeführt.

Unter Katalysatormasse wird hier, wie in der Fachwelt üblich, die beispielsweise in kg ausgedrückte Masse an aktivem Katalysatormaterial (d.h. ohne inerte Partikel bzw. Trägermaterialien und/oder inerte Binderanteile) verstanden, siehe auch untenstehende Erläuterungen zu den verwendeten Katalysatorbetten.

Die gewichtsbezogene stündliche Raumgeschwindigkeit (engl. Weight Hourly Space Velocity, WHSV) bezeichnet hier, wie in der Fachwelt üblich, die in kg ausgedrückte Menge an Gas (enthaltend die umzusetzenden Reaktanden inkl. möglicher Inertgaszusätze), die in einer Stunde über eine in kg ausgedrückte Menge an Katalysator geführt wird. Die WHSV entspricht also dem Quotienten aus der Massendurchflussrate (in kg/h) und der Masse des aktiven Katalysators (in kg) bzw. kg Gas/(kg Katalysator × h). Diese Definition entspricht der in WO 2015/082602 A1 verwendeten Konvention.

Unter der Lineargeschwindigkeit (engl. Linear Velocity) wird hier, wie ebenfalls fachüblich, die Geschwindigkeit eines Gasstroms (in cm/s) durch einen Reaktor bzw. ein Reaktionsrohr in Axialrichtung bzw. entlang einer Fließstrecke unter Berücksichtigung des für die Strömung verfügbaren Leerraumanteils des Katalysatorbetts verstanden. Die Lineargeschwindigkeit in m/h berechnet sich somit aus der Gasvolumendurchflussrate am Anfang der aktiven Katalysatorschüttung (in m³/h), geteilt durch die innere Querschnittsfläche des Reaktionsrohrs (in m²) und zusätzlich durch den mittleren Leerraumanteil im Katalysatorbett (dimensionslos). Diese Definition entspricht der in WO 2015/082598 A1 verwendeten Konvention. Die Umrechnung der Lineargeschwindigkeit von der Einheit m/h auf die für Zahlenangaben in dieser Anmeldung verwendete Einheit cm/s erfolgt mittels üblicher mathematischer Umrechnung.

Der Begriff Rohrbündelreaktor (engl. Shell-and-Tube Reactor) bezeichnet einen chemischen Reaktor, in dem eine exotherme Reaktion (hier die oxidative Dehydrierung) in der Gasphase durchgeführt wird. Das umzusetzende Gasgemisch wird dabei mithilfe eines Katalysators in mehreren fluidisch parallel geschalteten und typischerweise geraden und insbesondere aufrecht angeordneten Rohren (engl. Tubes) umgesetzt, die in einem Außenbehälter (engl. Shell) von einem oder mehreren Kühlmedien, insbesondere einer Salzschmelze, umflossen werden.

Unter dem Begriff des Katalysatorbetts wird hier eine Schüttung oder feste Struktur, die ein Katalysatormaterial und ggf. ein Inertmaterial als Träger oder Binder aufweist, verstanden. Des Weiteren können zumindest Teile eines Katalysatorbetts ggf. auch inerte Schüttungspartikel enthalten. Ist von mehreren Katalysatorbetten die Rede, können diese ohne oder mit zwischengeschalteten Zonen, insbesondere Inertzonen ohne Katalysatormaterial, in Flussrichtung hintereinander angeordnet sein. Generell entspricht die Flussrichtung eines Gases mit den umzusetzenden Reaktanden im vorliegend betrachteten Fall der Axialrichtung der Reaktionsrohre.

Nachfolgend ist mehrfach von einem Verhältnis einer Gesamtlänge eines oder mehrerer Katalysatorbetten zwischen den Enden eines oder mehrerer Reaktionsrohre zu einem Durchmesser jedes der Reaktionsrohre, kurz auch als "Gesamtlängen-Durchmesser-Verhältnis" bezeichnet, die Rede. Hierbei wird unter der Gesamtlänge des einen oder der mehreren Katalysatorbetten zwischen den Enden des einen oder der mehreren Reaktionsrohre die Länge verstanden, die in einem einzelnem Reaktionsrohr bzw. jedem Reaktionsrohr von dem oder den (aktiven) Katalysatorbetten eingenommen wird. Diese entspricht maximal der Gesamtlänge des jeweiligen Reaktionsrohrs zwischen seinen Enden, wenn keine katalysatorfreien Bereiche vorhanden sind. Für den genannten Wert werden stets (nur) jene Bereiche betrachtet, in denen ein aktives Katalysatormaterial in einem Massenanteil von mehr als 1, 2 oder 5% bereitgestellt ist. Bei mehreren Katalysatorbetten oder Katalysatorschichten werden deren Längen summiert. Die Länge oder Gesamtlänge der Reaktionsrohre entspricht bei einem typischerweise verwendeten, aufrechtstehenden Rohrbündelreaktor der Höhe bzw. Gesamthöhe der Reaktionsrohre. Unter dem Durchmesser wird der Innendurchmesser des jeweils betrachteten Reaktionsrohrs verstanden.

Als effektive Kühlfläche wird hier die Summe aller Reaktorrohrinnenflächen bezeichnet, die katalytisch aktive Schichten (mit den soeben erwähnten minimalen Masseanteilen an aktivem Katalysatormaterial) in den Reaktionsrohren umgeben. Reaktorrohrinnenflächen, die inerte Schichten oder leere Reaktorrohrabschnitte umgeben, werden hier somit nicht mit einberechnet.

Wie auch weiter unten unter Bezugnahme auf spezifische Beispiele erläutert, kann als Maß der Prozessintensität eines in einem Rohrbündelreaktor durchgeführten Verfahrens zur Herstellung von Ethylen durch oxidative Dehydrierung von Ethan der flächenspezifische Ethylenertrag verwendet werden, worunter hier der Ethylenertrag pro effektiver Kühlfläche (siehe unmittelbar zuvor) verstanden werden soll. Der spezifische Ethylenertrag wird insbesondere in kg Ethylen pro Stunde und Quadratmeter effektiver Kühlfläche mit der Einheit kg/(h × m²) ausgedrückt. Dieses Maß ist besonders aussagekräftig, da die insgesamt im Reaktor bereitgestellte Kühlfläche maßgeblich für den baulichen Aufwand und somit die Investitionskosten des Reaktors ist. Mit einem höheren flächenspezifischen Ethylenertrag, also einer höheren Prozessintensität im hier verstandenen Sinne, kann somit eine höhere Produktionsleistung bei gleichbleibender installierter Kühlfläche erreicht werden, was zu einer gewünschten Prozessintensivierung führt und sich positiv auf die Wirtschaftlichkeit der Produktionsanlage auswirkt. Mit dem Begriff der Prozessintensivierung wird die Erhöhung der Prozessintensität bezeichnet.

### Merkmale und Vorteile der Erfindung

Überraschenderweise wurde im Rahmen der vorliegenden Erfindung erkannt, dass für großtechnische Reaktoren unter bestimmten Bedingungen bei hoher Stabilität und Sicherheit der Prozessführung eine deutliche Prozessintensivierung gegenüber dem Stand der Technik erreicht werden kann.

Die in der erwähnten US 9,963,412 B2 angeführten Beispiele weisen Werte von weniger als 100 cm/s für die Lineargeschwindigkeit aus. Die begleitende Entstehung von Essigsäure wird nicht erwähnt. Die genannte Schrift lässt somit keine Schlüsse auf die für die Ethylen-/Essigsäure-Ko-Produktion besonders vorteilhaften Geschwindigkeitsbereiche zu. Hinzu kommt, dass aus den in dieser Druckschrift genannten Beispielen im Labormaßstab keine Rückschlüsse auf bevorzugte Geschwindigkeitsbereiche für Reaktoren im großtechnischen Maßstab möglich sind. Ähnliches gilt beispielsweise für die US 10,017,432 B2, in der lediglich auf Basis von Laborversuchen bevorzugte Bereiche von gewichtsbezogenen stündlichen Raumgeschwindigkeiten angegeben werden.

In der Tat unterscheiden sich Laborreaktoren deutlich von den in großtechnischen Anlagen eingesetzten Reaktoren, insbesondere hinsichtlich des erwähnten Gesamtlängen-Durchmesser-Verhältnisses der einen Reaktionszone oder der mehreren Reaktionszonen. Die in den soeben genannten Schriften gezeigten Beispiele weisen dabei Verhältnisse von unter 100 auf. Im Rahmen der vorliegenden Erfindung werden jedoch Reaktoren mit den zuvor genannten, für großtechnische Anwendungen relevanten Gesamtlängen-Durchmesser-Verhältnissen der einen Reaktionszone oder der mehreren Reaktionszonen betrachtet.

Wie auch weiter unten unter Bezugnahme auf die Figuren erläutert, in denen teilweise aus dem Stand der Technik ermittelte Daten erfindungsgemäßen Daten gegenübergestellt sind, führen im Stand der Technik vorgeschlagene Verfahren und dort angegebene Beispiele zu spezifischen Ethylenerträgen (siehe oben) von ungefähr 2 kg/(h × m²) bei den genannten vergleichsweise geringen Gesamtlängen-Durchmesser-Verhältnissen von weniger als 100.

Ein ähnliches Maß der Prozessintensivierung kann auch für Reaktoren im Industriemaßstab und Gesamtlängen-Durchmesser-Verhältnissen der einen Reaktionszone oder der mehreren Reaktionszonen von 200 bis 300 erreicht werden, wie beispielsweise auch in der WO 2018/115416 A1 offenbart und dort mit Versuchsergebnissen belegt. Gleiches gilt für Pilotversuche im Stand der Technik. In der WO 2018/115416 A1 wurde dabei auch gezeigt, dass durch Erhöhung der Durchströmungsrate und entsprechender Temperaturanpassung eine Erhöhung der spezifischen Ethylenausbeute auf Werte von ca. 2,5 kg/(h × m²) erreicht werden konnten. Es zeigt sich jedoch, dass der dabei erreichte Anstieg verhältnismäßig flach verläuft, da bei höherer Durchströmung die maximale Ethankonversion verringert werden muss, weil anderenfalls ein Durchgehen des Reaktors nicht vermieden werden kann. Durch diese thermische Begrenzung würde eine weitere Erhöhung der Ethanlast daher zu einer verhältnismäßig immer kleiner werdenden Erhöhung der Prozessintensität führen.

In der WO 2019/243480 A1 wurde gezeigt, dass durch Verwendung eines dreilagigen Katalysatorbetts eine deutliche Prozessintensivierung gegenüber einem einlagigen Katalysatorbett erreicht werden kann. Auch hier werden dabei Werte von bis zu 2,5 kg/(h × m²) für den spezifischen Ethylenertrag erreicht. Wiederum wird zu weiteren Details auf die Erläuterungen zu den Figuren verwiesen.

Bei einer Auswertung der Daten aus den erwähnten Druckschriften ergibt sich insgesamt, dass die für ähnliche Ethylenerträge benötigten Lineargeschwindigkeiten bei großtechnischen Reaktoren deutlich höher als bei Laborreaktoren liegen.

Die beispielsweise in der US 9,963,412 B2 angegebenen Werte sind daher nicht für den direkten Übertrag auf großtechnische Reaktoren geeignet. Auch zeigt sich, dass im Vergleich zwischen einem ein- und einem dreilagigen Katalysatorbett eine gleichwertige oder bessere Prozessintensität bei niedrigerer Lineargeschwindigkeit erreicht werden kann. Das Design des Katalysatorbetts ist somit insbesondere im großtechnischen Maßstab von großer Relevanz.

Ähnliches wie für die lineare Geschwindigkeit gilt für die gewichtsbezogene stündliche Raumgeschwindigkeit, wie ebenfalls in Bezugnahme auf die Figuren näher erläutert, da das gleiche Niveau der Prozessintensität in großtechnischen Reaktoren bei deutlich geringeren gewichtsbezogenen stündlichen Raumgeschwindigkeiten als im Labor erreicht werden kann. Im Übrigen zeigt sich auch bei Gegenüberstellung der Daten aus der US 10,017,432 B2 und der US 9,963,412 B2, dass kein eindeutiger Zusammenhang zwischen der gewichtsbezogenen stündlichen Raumgeschwindigkeit und der erreichbaren Prozessintensität erstellt werden kann.

Die Erfindung geht insgesamt von einem Verfahren zur Herstellung von Ethylen durch oxidative Dehydrierung von Ethan aus, bei dem ein Rohrbündelreaktor mit Reaktionsrohren verwendet wird, die sich zwischen einem ersten Ende und einem zweiten Ende erstrecken, wobei in jedem der Reaktionsrohre ein oder mehrere Katalysatorbetten angeordnet sind und ein Verhältnis einer Gesamtlänge des einen oder der mehreren Katalysatorbetten zwischen dem ersten Ende und dem zweiten Ende zu einem Durchmesser jedes der Reaktionsrohre (also das erwähnte Gesamtlängen-Durchmesser-Verhältnis der Reaktionszone oder Reaktionszonen) in jedem der Reaktionsrohre einen Wert zwischen 150 und 400, insbesondere zwischen 200 und 280, aufweist. Die vorliegende Erfindung bezieht sich also ausdrücklich auf Rohrbündelreaktoren im großtechnisch relevanten Maßstab.

Erfindungsgemäß wird der Rohrbündelreaktor mit einer Lineargeschwindigkeit von 250 300, 400 oder 500 cm/s bis 800 cm/s betrieben und das eine oder die mehreren Katalysatorbetten werden ferner derart ausgestaltet, dass ein Verhältnis von aktiver Katalysatormasse zu wirksamer Kühlfläche in einem Bereich zwischen 1,5 und 5 kg/m², insbesondere zwischen 2 und 4 kg/m², liegt.

Bevorzugt handelt es sich bei dem erfindungsgemäß eingesetzten Rohrbündelreaktor um einen Rohrbündelreaktor mit festen Katalysatorbetten im jeweils einzelnen Reaktionsrohr. Um die Gesamtwirtschaftlichkeit sowie eine erhöhte Betriebssicherheit zu erreichen, sind die einzelnen Reaktionsrohre dabei mit mehreren Katalysatorbetten, insbesondere mit 1, 2, 3, 4, 5 Katalysatorbetten unterschiedlicher Aktivität und/oder Zusammensetzung ausgestattet. Die Kühlung bzw. Beheizung erfolgt mit einem geeignetem Kühlmedium, insbesondere einem Thermoöl oder vorzugsweise einer Salzschmelze, wobei diese im Gleich- oder Gegenstrom zur Strömungsrichtung des Reaktionseinsatzes geführt wird, bevorzugt im Gegenstrom.

Unterschiedliche Reaktionszonen können zum einen durch unterschiedlich aktive Katalysatorlagen (mit zunehmender Aktivität in Flussrichtung des Reaktionseinsatzstromes) und/oder mit unterschiedlich gekühlten Zonen, d.h. unterschiedlichen Kühlmittel- bzw. Salzschmelzekreisläufen (diese jeweils ggf. unterschiedlich im Gleich- oder Gegenstrom) erreicht werden. Beispielsweise kann der Reaktor wie in der WO 2019/243480 A1 angegeben ausgestaltet sein.

Typische Betriebsbedingungen sind beispielsweise in der WO 2018/115416A1 und der WO 2018/115418 A1 sowie in den untenstehenden Tabellen 1A und 1B angegeben.

Durch die erfindungsgemäß vorgeschlagene vorteilhafte Verknüpfung von Reaktionsbedingungen und baulicher Ausgestaltungen wird erstmals für großtechnisch betriebene Reaktoren eine Kombination von Designgrößen, nämlich des Seitenverhältnisses des Katalysatorbetts und des Verhältnisses der Katalysatormasse zur Kühlfläche und Betriebsparametern, nämlich insbesondere der Lineargeschwindigkeit, beschrieben, die eine deutliche Steigerung der Prozessintensität bei der oxidativen Dehydrierung von Ethan ermöglichen. Erfindungsgemäß wurde erkannt, dass nur in einem sehr bestimmten, von mehreren Parametern aufgespannten Bereich eine signifikante Verbesserung im Sinne einer Prozessintensivierung möglich ist.

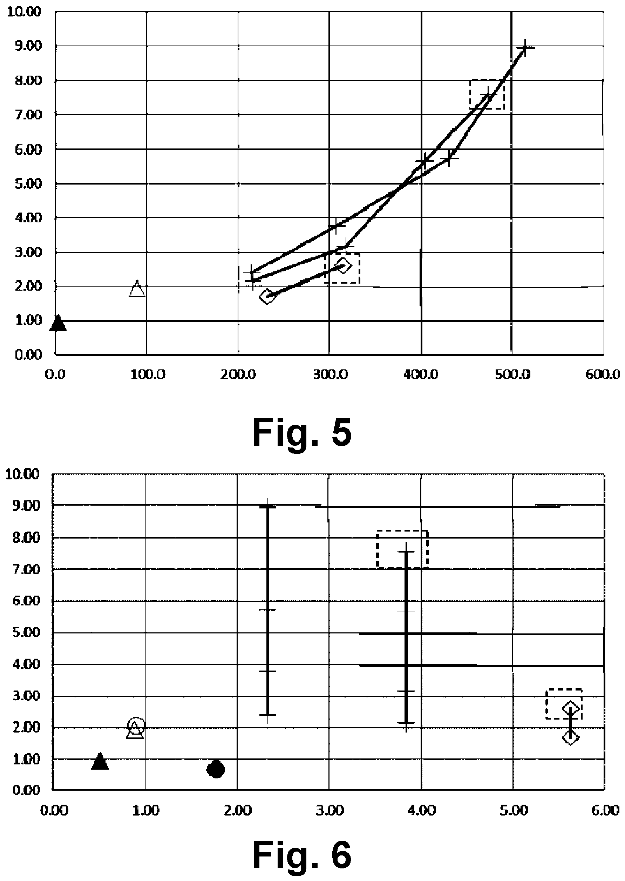

Wie weiter unten unter Bezugnahme auf die Figuren (siehe insbesondere Figuren 5 und 6 und zugehörige Erläuterungen) dargestellt, wird nur für den beanspruchten mittleren Bereich des Verhältnisses von Katalysatormasse zu Kühlfläche durch die Erhöhung der Lineargeschwindigkeit eine starke Prozessintensivierung erreicht. Bei den im Stand der Technik gewählten Bedingungen mit einer hohen spezifischen Katalysatorbeladung pro Kühlfläche besteht dagegen kein Potential für weitere Ausbeutesteigerungen.

Überraschend ist insbesondere die Tatsache, dass in erfindungsgemäßen Ausführungen selbst für sehr hohe spezifische Ethylenerträge kein thermisches Durchgehen zu erwarten ist. Für alle Betrachtungen, denen ein Rohrdurchmesser von 20 mm zugrunde liegt, wird stets eine Ethankonversion von mehr als 50% erreicht, bei einer maximalen Temperaturüberhöhung im Hotspot, d.h. dem wärmsten Punkt im jeweiligen Reaktionsrohr, von weniger als 45 K, bezogen auf eine Austrittstemperatur des verwendeten Kühlmittels, hier insbesondere einer Salzschmelze.

Bei einem Reaktor mit einem Rohrdurchmesser von 26 mm ergeben sich im Allgemeinen höhere Temperaturanstiege im Katalysatorbett, die beim Punkt mit der höchsten Reaktorlast auch zu einem thermischen Durchgehen führen könnte, falls die Temperatur des Kühlmittels weiter erhöht wird. Es zeigt sich also, dass erst durch ein geeignetes Katalysatorbettdesign, mit moderaten Verhältnissen von Katalysatormasse zu Kühlfläche, das Potential einer Erhöhung der Lineargeschwindigkeit in ausreichendem Umfang ausgenutzt werden kann.

Die Wirkung der erfindungsgemäßen Reaktionsführung lässt sich anhand der nachfolgend erläuterten Einflüsse anschaulich beschreiben.

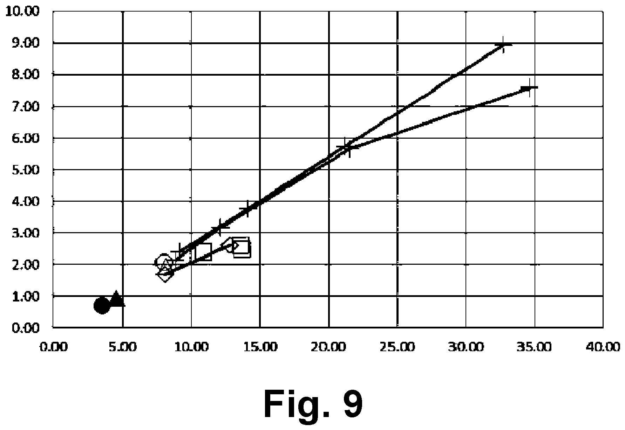

Bei höherer Lineargeschwindigkeit kann der ODHE-Reaktor bei höherer Reaktionstemperatur, jedoch weiterhin moderaten Temperaturen von max. 450 bis 500 °C betrieben werden. Die höhere Reaktionstemperatur ist maßgeblich entscheidend für eine Konversionserhöhung bei gleichzeitig gesteigerter Ethylenselektivität. Dies führt einerseits zu steilen Anstiegen des spezifischen Ethylenertrags bei zunehmender Einsatzbeladung (siehe insbesondere Figur 9 und zugehörige Erläuterungen). Ohne zu sehr an die Theorie gebunden zu sein, wird eine Erklärung hierfür in WO 2019/243480 A1 gegeben. Dieser Effekt konnte im Rahmen der vorliegenden Erfindung ebenfalls nachvollzogen werden.

Bei moderater durchschnittlicher Katalysatorbeladung pro Kühlfläche wird die Abfuhr von Wärme insbesondere im Bereich des Hotspots begünstigt, sodass eine höhere Stabilität der Reaktion in Bezug auf thermisches Durchgehen erreicht wird. Bei großtechnischen Produktionsraten muss das Verhältnis jedoch ein Mindestmaß von 1,5, bevorzugt 2 übersteigen, um einen wirtschaftlichen Betrieb zu gewährleisten.

Eine hohe Lineargeschwindigkeit lässt sich bevorzugt durch Erhöhung des Gesamtvolumenstromes bei unveränderter Zusammensetzung am Katalysatorbetteintritt oder aber insbesondere durch die Wahl eines Reaktionsrohres mit einem möglichst geringen Durchmesser erreichen. Letzterer muss aber dennoch groß genug sein, dass eine ausreichend große Menge an kommerziellen Katalysatorformkörpern im Reaktionsrohr bzw. über den Rohrquerschnitt gepackt werden können, um den Anteil an Bypassströmung so gering wie möglich und die Katalysatornutzung möglichst hoch zu halten.

Ein zu geringer Rohrquerschnitt würde bewirken, dass auch der wirksame Durchmesser der Katalysatorformkörper deutlich verringert werden müsste, wodurch der Druckverlust über das jeweilige Rohr inakzeptabel hoch ausfallen würde.

Ein zu großer Rohrquerschnitt bewirkt, dass zum Erreichen einer hohen Lineargeschwindigkeit die Gaslast und somit die Katalysatorbelastung zu stark erhöht werden müsste, um den Ethanumsatz in einem gesamtwirtschaftlich sinnvollen Rahmen zu halten. Damit verbunden ist eine sehr starke Wärmetönung der Reaktion und ebenso eine, aufgrund eines großen Rohrquerschnitts, geringere Wärmeabfuhr aus dem Rohr ins Kühlmedium des Reaktors. Dadurch bestünde die erhöhte Gefahr eines thermischen Durchgehens. Daher kommt ein gekühlter Rohrbündelreaktor zum Einsatz, wobei der Innendurchmesser des Einzelrohres vorzugsweise 10 bis 32 mm, insbesondere 12 bis 26 mm, insbesondere 15 bis 21 mm, beträgt.

Bei Bedarf kann auch eine Erhöhung der Lineargeschwindigkeit durch eine Erhöhung des Gesamteinsatzgasstromes bei veränderter Zusammensetzung am Katalysatorbettanfang erreicht werden. Um eine zu große Wärmetönung der Reaktion zu vermeiden, kann die Erhöhung der Gaslast insbesondere durch Verdünnen mit einem Inertgas erreicht werden. Als Inertgas kann hierbei ein Gas oder ein Gasgemisch aus der Gruppe der Edelgase (also He, Ne, Ar, Kr oder Xe), Kohlendioxid, Stickstoff oder Methan verwendet werden. Allerdings bieten sich für einen technischen Prozess besonders bevorzugt Kohlendioxid, Stickstoff oder Methan an. Insbesondere kann eine hohe Lineargeschwindigkeit durch Kombination einer Eduktgasverdünnung und der Verwendung eines möglichst geringen Reaktionsrohrquerschnitts erreicht werden. Eine weitere Erhöhung des Wasser(dampf)anteils im Eduktgas über den für die Katalysatorstabilität benötigten Anteil hinaus, wie in der WO 2018/115418 A1 beschrieben, sollte hierbei vermieden werden, um den erfindungsgemäß erzielbaren Selektivitätsvorteil zu Ethylen zu erhalten.

Durch die Erfindung wird der wirtschaftliche Nutzen der ODHE-Technologie nochmals deutlich verstärkt, da bei gleicher Anlagenkapazität bzgl. des Produkts Ethylen, die Reaktoren deutlich kleiner (hinsichtlich Anzahl und Länge der einzelnen Rohre eines Rohrbündelreaktors, vgl. Tabellen 1A und 1B) und mit reduzierter Wärmeaustauschfläche gebaut werden können, was eine höhere Prozessintensität bewirkt. Des Weiteren wird auch eine technische Umsetzung für größere Anlagenkapazitäten (bezogen auf Ethylenprodukt) erleichtert.

In einer besonders vorteilhaften Ausgestaltung der Erfindung wird der Rohrbündelreaktor mit einer gewichtsbezogenen stündlichen Raumgeschwindigkeit von mehr als 3 oder 5 kg und weniger als 20 kg Einsatz pro Stunde und kg Katalysator betrieben. Die spezifischen Vorteile dieser Betriebsweise und der auch nachfolgend angegebenen Aspekte hiervon ergeben sich insbesondere in Kombination mit den weiteren erfindungsgemäßen und gemäß vorteilhafter Ausgestaltungen vorgesehenen Merkmalen und werden nachfolgend auch unter Bezugnahme auf die beigefügten Figuren erläutert.

Die erfindungsgemäß in dem Rohrbündelreaktor eingesetzte Lineargeschwindigkeit beträgt vorteilhafterweise mehr als 250, 300, 400 oder 500 cm/s, jedoch weniger als 800 cm/s.

Der Rohrbündelreaktor wird vorteilhafterweise so betrieben, dass eine Maximaltemperatur in dem einen oder den mehreren Katalysatorbetten weniger als 500 oder 450 °C beträgt.

In dem Rohrbündelreaktor werden vorteilhafterweise in dem einen oder den mehreren Katalysatorbetten Füllkörper verwendet, die aus Ringen, insbesondere Raschig-Ringen, Pellets, insbesondere zylindrischen Pellets, und Extrudaten oder Kombinationen hiervon ausgewählt sind. Die Extrudate können insbesondere eine hohe Oberfläche aufweisen, die durch entsprechende geometrische Formen wie z.B. sogenannten Kleeblattstrukturen, erreicht werden kann.

In dem erfindungsgemäßen Verfahren werden vorteilhafterweise mehrere Katalysatorbetten mit unterschiedlicher Katalysatoraktivität verwendet, wie beispielsweise in der WO 2019/243480 A1 offenbart und bereits oben erwähnt.

Eine besonders vorteilhafte Ausgestaltung der vorliegenden Erfindung umfasst, in Abhängigkeit von einer detektierten Katalysatoraktivität in dem Rohrbündelreaktor, beispielsweise festgestellt über eine gemessene Temperatur, eine Wasser(dampf)zugabe in den Einsatzstrom zum Rohrbündelreaktor, nachfolgend auch als Wassereinspeisung bezeichnet, zu dosieren, wie auch in der WO 2018/115418 A1 offenbart. Eine Dosierung der Wassereinspeisung kann, wie in der WO 2018/115416 A1 offenbart, auch zur Regelung des Produktverhältnisses zwischen Ethylen und Essigsäure verwendet werden, insbesondere als Funktion des ermittelten Wasserpartialdrucks im Produktgas am Reaktoraustritt.

Ein Verfahren gemäß einer besonders bevorzugten Ausgestaltung der Erfindung umfasst, dass der Rohrbündelreaktor mit einem oder mehreren Kühlmedien durchströmt wird und dabei unterschiedliche Bereiche des Rohrbündelreaktors insbesondere in unterschiedlichem Ausmaß gekühlt werden.

Eine Anlage zur Herstellung von Ethylen durch oxidative Dehydrierung von Ethan, die einen Rohrbündelreaktor mit Reaktionsrohren aufweist, die sich zwischen einem ersten Ende und einem zweiten Ende erstrecken ist ebenfalls Gegenstand der Erfindung. In jedem der Reaktionsrohre sind ein oder mehrere Katalysatorbetten angeordnet und ein Verhältnis einer Gesamtlänge des einen oder der mehreren Katalysatorbetten zwischen dem ersten Ende und dem zweiten Ende zu einem Durchmesser jedes der Reaktionsrohre in jedem der Reaktionsrohre weist einen Wert zwischen 150 und 400 auf. Erfindungsgemäß ist der Rohrbündelreaktor der erfindungsgemäßen Anlage für einen Betrieb mit einer Lineargeschwindigkeit von 250 bis 800 cm/s eingerichtet und das eine oder die mehreren Katalysatorbetten sind derart ausgestaltet, dass ein Verhältnis von aktiver Katalysatormasse zu wirksamer Kühlfläche in einem Bereich zwischen 1,5 und 5 kg/m² liegt.

Zu der erfindungsgemäß bereitgestellten Anlage und ihren Merkmalen sei auf die obigen Erläuterungen bezüglich des erfindungsgemäßen Verfahrens ausdrücklich verwiesen, da diese eine entsprechende Anlage in gleicher Weise betreffen. Entsprechendes gilt insbesondere für eine Ausgestaltung einer entsprechenden Anlage, die vorteilhafterweise zur Ausführung eines entsprechenden Verfahrens in einer beliebigen Ausgestaltung eingerichtet ist.

Eine Ausgestaltung des erfindungsgemäßen Verfahrens umfasst, dass die oxidative Dehydrierung bei einer Temperatur des Katalysators in einem Bereich zwischen 240 und 500 °C, bevorzugt zwischen 280 und 450 °C, durchgeführt wird.

In einer weiteren Ausgestaltung werden für die oxidative Dehydrierung ein oder mehrere Reaktoren verwendet und der Gesamtdruck des Reaktionseinsatzstromes am Eintritt des oder der Reaktoren wird in einem Bereich zwischen 1 und 10 bar (abs.), bevorzugt zwischen 2 und 7 bar (abs.) gewählt.

Der vorteilhaft eingesetzte Wasseranteil des Reaktionseinsatzstroms liegt in einem Bereich zwischen 5 und 95 vol.-%, insbesondere 10 und 50 vol.-%, insbesondere 14 und 35 vol.-%. Das molare Verhältnis von Wasser zu Ethan im Reaktionseinsatzstrom kann dabei mindestens 0,23 betragen.

Der verwendete Katalysator kann zumindest die Elemente Molybdän, Vanadium, Niob und optional Tellur, insbesondere in Form eines Mischoxids, enthalten.

Die Erfindung wird nachfolgend unter Bezugnahme spezifische erfindungsgemäße Beispiele und Vergleichsbeispiele mit zugehörigen Figuren sowie auf eine Figur, die eine Ausgestaltung der Erfindung veranschaulicht, weiter erläutert.

### Kurze Beschreibung der Zeichnungen

Figuren 1 bis 9 veranschaulicht erfindungsgemäß ermittelte Daten und nicht erfindungsgemäße Vergleichsdaten in Form von Diagrammen.
Figur 10 veranschaulicht eine Anlage zur Herstellung von Ethylen durch oxidative Dehydrierung von Ethan gemäß einer Ausführungsform der Erfindung.

### Ausführliche Beschreibung der Zeichnungen

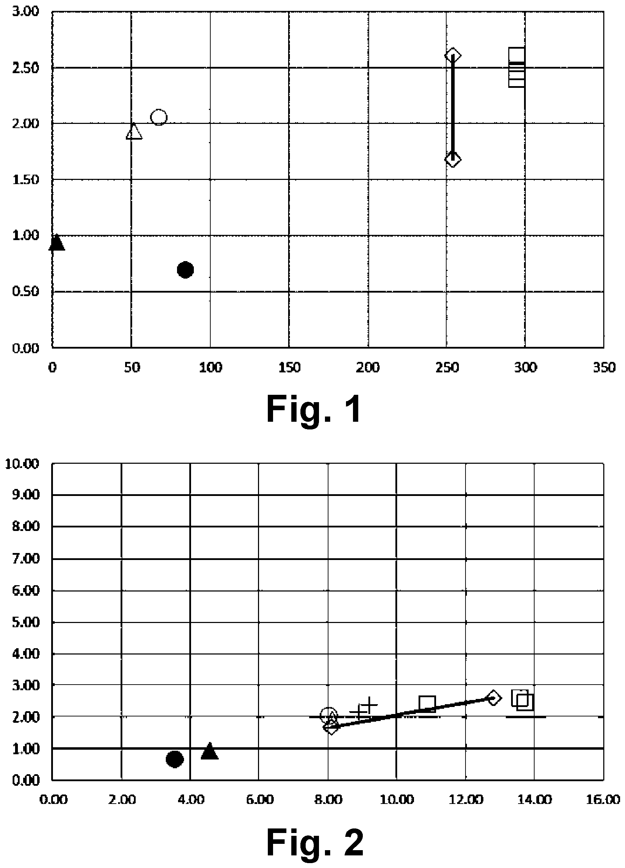

Figuren 1 bis 9 veranschaulicht erfindungsgemäß erhaltene Daten und nicht erfindungsgemäße Vergleichsdaten in Form von Diagrammen.

Wie erwähnt, weisen im Stand der Technik verwendete Laborreaktoren, wie sie beispielsweise gemäß US 10,017,432 B2 und US 9,963,412 B2 eingesetzt werden, Seitenverhältnisse von weniger als 100 auf. Im Rahmen der vorliegenden Erfindung stehen jedoch Reaktoren mit einem Seitenverhältnis zwischen 150 und 400 im Fokus, der für großtechnische Anwendungen besonders relevant ist.

Figur 1 veranschaulicht Daten, die auf Grundlage der Offenbarung der mehrfach erwähnten US 10,017,432 B2 (nachfolgend als Druckschrift 1 bezeichnet) rechnerisch ermittelt wurden, sowie ferner Daten, die auf Grundlage der Offenbarung der mehrfach erwähnten US 9,963,412 B2 (Druckschrift 2) ermittelt wurden. Ferner sind Daten, die auf Grundlage der erwähnten WO 2020/074750 A1 (Druckschrift 3) ermittelt wurden, dargestellt. Die Daten gemäß Druckschrift 1 sind in Form von Kreisen dargestellt, wobei ausgefüllte Kreise Daten aus Vergleichsbeispielen bezeichnen. Daten gemäß Druckschrift 2 sind in Form von Dreiecken veranschaulicht, wobei ausgefüllte Kreise Daten aus Vergleichsbeispielen bezeichnen. Auf einer Kante liegende Quadrate entsprechen den Daten gemäß Druckschrift 3 bzw. der dort offenbarten Pilotanlage. Auf Grundlage der WO 2018/115416 A1 (Druckschrift 4) ermittelte Daten sind als Rauten bzw. schräggestellte Quadrate dargestellt und mit einer Linie verbunden.

Das Diagramm der Figur 1 und alle nachfolgenden Diagramme geben auf der Vertikalachse als Maß der Prozessintensivierung den oben mehrfach erwähnten spezifischen Ethylenertrag, d.h. den Ethylenertrag pro effektiver Kühlfläche in kg/(h × m²), an. Auf der Horizontalachse ist in Figur 1 das erläuterte Gesamtlängen-Durchmesser-Verhältnis in dimensionslosen Einheiten angegeben.

Aus Figur 1 wird ersichtlich, dass die positiven Beispiele aus den laborbasierten Daten der erwähnten Druckschriften 1 und 2 (nicht ausgefüllte Kreise und Dreiecke) bei kleinen Längen-Durchmesser-Verhältnissen von weniger als 100 zu spezifischen Ethylenerträgen von ca. 2 kg/(h × m²) führen.

Ein nur geringfügig erhöhtes Maß der Prozessintensität mit spezifischen Ethylenerträgen von ca. 2,5 kg/(h × m²) kann auch für Reaktoren im Industriemaßstab und Seitenverhältnissen von 200 bis 300 erreicht werden, wie beispielsweise auch aus den Daten der Druckschrift 4 (schräggestellte Quadrate) und der Druckschrift 3 (liegende Quadrate) belegt.

Figur 2 veranschaulicht neben den bereits zu Figur 1 erläuterten Daten ferner Daten, die gemäß der WO 2019/243480 A1 (Druckschrift 5) bei Annahme unterschiedlicher Reaktionsrohrdurchmesser erhalten wurden. Die Daten sind in Form senkrecht stehender Kreuze veranschaulicht, wobei der Datenpunkt links unten einem Reaktionsrohrdurchmesser von 26 mm, der Datenpunkt rechts oben einem Reaktionsrohrdurchmesser von 20 mm entspricht. In Druckschrift 5 wurde gezeigt, dass durch Verwendung eines dreilagigen Katalysatorbetts eine deutliche Prozessintensivierung gegenüber einem einlagigen Bett erreicht werden kann. Die entsprechenden Datenpunkte wurden unter diesen Voraussetzungen ermittelt. Die Vertikalachse entspricht der Vertikalachse gemäß Figur 1. Auf der Horizontalachse ist die Beladung mit Einsatz pro Kühlfläche in kg/(h × m²) angegeben, d.h. der Quotient zwischen Einsatzgasmassenstrom und effektiver Kühlfläche.

Wie sich hierbei auch aus den Daten gemäß Druckschrift 4 (schräggestellte Quadrate) ergibt, kann durch eine Erhöhung der Durchströmungsrate und entsprechender Temperaturanpassung eine Erhöhung des spezifischen Ethylenertrags auf besagte Werte von ca. 2,5 kg/(h × m²) erreicht werden. Auch gemäß Druckschrift 5 (senkrechte Kreuze) können Werte von bis zu 2,5 kg/(h × m²) für den spezifischen Ethylenertrag erreicht werden. Es zeigt sich jedoch insbesondere, dass der dabei erreichte Anstieg verhältnismäßig flach verläuft, da bei höherer Durchströmung (Versuchspunkt 56 gemäß Druckschrift 4) die maximale Ethankonversion niedriger lag, da sonst ein Durchgehen des Reaktors nicht vermieden werden konnte. Durch diese Begrenzung würde eine weitere Erhöhung der Ethanlast daher zu einer verhältnismäßig immer kleiner werdenden Erhöhung der Prozessintensivierung führen.

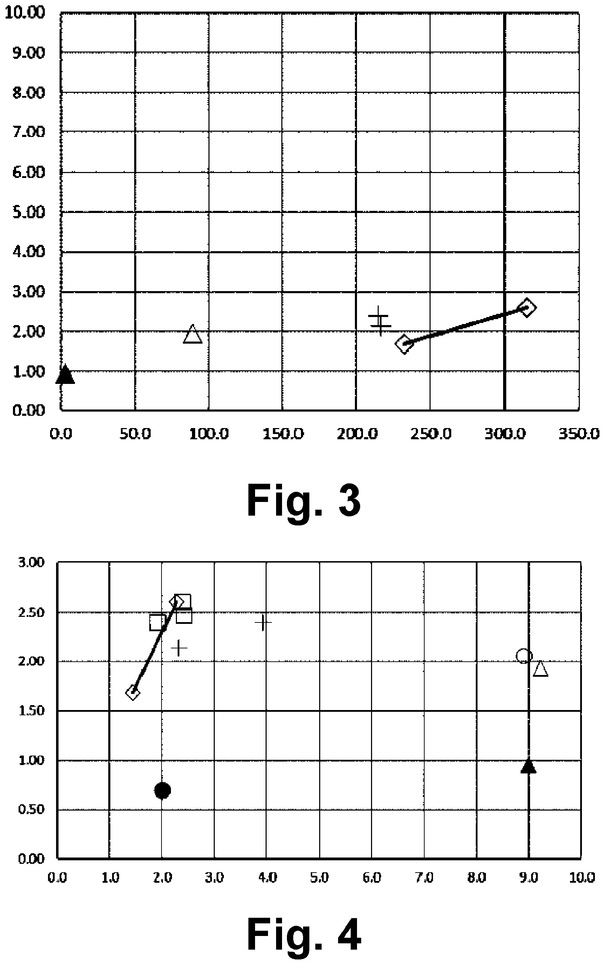

Figur 3 veranschaulicht einen Teil der bereits zu Figur 2 erläuterten Daten, wobei auf der Horizontalachse die Lineargeschwindigkeit in cm/s angegeben ist. Mit Kreuzen sind wiederum die Daten gemäß Druckschrift 5 veranschaulicht, wobei der Datenpunkt links oben einem Reaktionsrohrdurchmesser von 20 mm, der Datenpunkt rechts unten einem Reaktionsrohrdurchmesser von 26 mm entspricht.

Wie aus Figur 3 ersichtlich, liegen die für ähnliche Ethylenerträge benötigten linearen Geschwindigkeiten bei großtechnischen Reaktoren deutlich höher als bei Laborreaktoren. Für die Ermittlung der Lineargeschwindigkeit wurde die in der Druckschrift 2 angegebene Definition verwendet. Die in der Druckschrift 2 angegebenen Werte sind nicht für direkten Übertrag auf großtechnische Reaktoren geeignet. Auch zeigt sich, dass im Vergleich von ein- zu dreilagigem Bett (senkrechte Kreuze gemäß Druckschrift 5) eine gleichwertige oder bessere Prozessintensität bei niedrigerer Lineargeschwindigkeit erreicht werden kann. Das Design des Katalysatorbetts ist somit, wie bereits erläutert, insbesondere im großtechnischen Maßstab von großer Relevanz.

Figur 4 veranschaulicht die bereits zu Figur 2 und 3 erläuterten Daten, wobei auf der Horizontalachse die WHSV in kg Gas/(kg Katalysator × h) angegeben ist. Mit Kreuzen sind wiederum die Daten gemäß Druckschrift 5 veranschaulicht, wobei der Datenpunkt links unten einem Reaktionsrohrdurchmesser von 26 mm, der Datenpunkt rechts oben einem Reaktionsrohrdurchmesser von 20 mm entspricht.

Aus Figur 4 ergibt sich, dass eine Unterscheidung zwischen labortechnischen und großtechnischen Reaktoren wie für die lineare Geschwindigkeit gemäß Figur 3 auch für die WHSV gilt, da das gleiche Niveau der Prozessintensivität in großtechnischen Reaktoren bei deutlich geringeren WHSV als im Labor erreicht werden kann. Im Übrigen zeigt sich auch bei Gegenüberstellung der Daten aus den Druckschriften 1 und 2 (Kreise und Dreiecke), dass kein eindeutiger Zusammenhang zwischen WHSV und erreichbarer Prozessintensität erstellt werden kann.

Die vorliegende Erfindung erlaubt nun in erfindungsgemäßen Ausführungsformen für großtechnische Reaktoren eine weitergehende Prozessintensivierung, bei größtmöglicher Stabilität und Sicherheit der Prozessführung.

Figur 5 veranschaulicht dabei einen Teil der zuvor zu den Figuren 1 bis 4 erläuterten Daten in einem Diagramm, in dem die Lineargeschwindigkeit in cm/s auf der Horizontalachse aufgetragen sind.

Wiederum sind in Form von senkrechten Kreuzen die erfindungsgemäß erhaltenen Daten mit einem Rohrdurchmesser von 26 mm (bei einem Wert von ca. 2,2 auf der Vertikalachse beginnender und bei einem Wert von ca. 7,5 auf Vertikalachse endender Graph) und 20 mm (bei einem Wert von ca. 2,5 auf der Vertikalachse beginnender und bei einem Wert von ca. 8,9 auf der Vertikalachse endender Graph) veranschaulicht. An den mit gestrichelten Rechtecken umgebenen Positionen besteht die Gefahr eines thermischen Durchgehens in verstärkter Weise.

Figur 6 veranschaulicht erneut die in Figur 5 gezeigten Daten in einem Diagramm, in dem die spezifische Katalysatorbeladung pro Kühlfläche in kg/m² auf der Horizontalachse aufgetragen ist.

Wiederum sind in Form von senkrechten Kreuzen die erfindungsgemäß erhaltenen Daten mit einem Rohrdurchmesser von 26 mm (bei höheren Werten auf der Horizontalachse) und 20 mm (bei geringeren Werten auf der Horizontalachse) veranschaulicht. An den mit gestrichelten Rechtecken umgebenen Positionen besteht die Gefahr eines thermischen Durchgehens in verstärkter Weise.

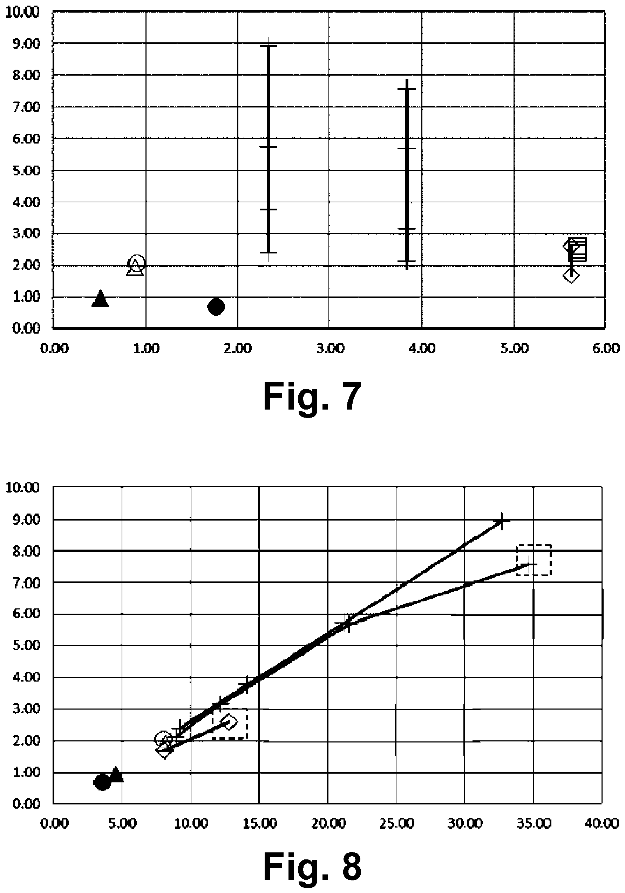

In Figur 7 sind die in Figur 6 veranschaulichten Daten um die anhand von Druckschrift 3 erhaltenen Daten (liegende Quadrate) ergänzt, bei ansonsten gleicher Darstellung.

Aus der Zusammenschau insbesondere der Figuren 5 und 6 wird ersichtlich, dass nur für den erfindungsgemäß beanspruchten mittleren Bereich des Verhältnisses Katalysatormasse zu Kühlfläche durch Erhöhung der Lineargeschwindigkeit eine starke Prozessintensivierung erreicht werden kann. Bei Betrachtung von Figur 7 zeigt sich zudem, dass auch für die gemäß Druckschrift 3 gewählten Bedingungen mit einer hohen spezifischen Katalysatorbeladung pro Kühlfläche kein Potential für weitere Ausbeutesteigerungen aufgezeigt wurde.

Überraschend ist insbesondere die Tatsache, dass in erfindungsgemäßen Ausführungen selbst für sehr hohe spezifische Ethylenerträge kein thermisches Durchgehen zu erwarten ist. Für alle Betrachtungen mit einem Reaktionsrohr von 20 mm wird stets eine Ethankonversion von mehr als 50% erreicht, bei einer maximalen Temperaturüberhöhung im Hotspot von weniger als 45 K gegenüber der Salzaustrittstemperatur. Bei einem Reaktionsrohr von 26 mm ergeben sich im Allgemeinen höhere Temperaturanstiege im Katalysatorbett, die ausgehend vom Punkt mit der höchsten Reaktorlast auch zu einem thermischen Durchgehen führen kann, falls die Salztemperatur weiter erhöht wird.

Es zeigt sich also, dass erst durch geeignetes Katalysatorbettdesign, mit moderaten Verhältnissen von Katalysatormasse zu Kühlfläche, das Potential einer Erhöhung der Lineargeschwindigkeit voll ausgenutzt werden kann.

Das zeigt sich auch bei Betrachtung der Figuren 8 und 9, die, im Fall von Figur 8 im Vergleich zu Figur 2, um die gemäß der Erfindung erhaltenen Daten (Kreuze, obere Datenpunkte entsprechen jeweils dem Rohrdurchmesser 20 mm) und im Fall von Figur 9 zusätzlich um die Daten gemäß Druckschrift 3 ergänzt sind. Für diese Ausführungen kann ein nahezu linearer Anstieg des spezifischen Ethylenertrags mit zunehmender Last bis in sehr hohe Bereiche erreicht werden. Dies ist insbesondere im Gegensatz zum beschriebenen recht flachen Anstieg für das einlagige Bett mit hohem Verhältnis von Katalysatormasse zu Kühlfläche zu verzeichnen.

In den Tabellen 1A und 1B ist jeweils ein Vergleich der Reaktordaten hinsichtlich Reaktionsparameter und Produktperformance für jeweils einen Standardauslegungsfall (Basisfall) und mehrere Vergleichsfälle bei hoher Lineargeschwindigkeit für einen Rohrdurchmesser von 20 mm (Tabelle 1A) und 26 mm (Tabelle 1B) dargestellt. Als Katalysator wurde ein MoVNbTe-basiertes Mischoxid, das auf einem Träger in Form von Ringen in den Reaktor eingefüllt wird, verwendet. Das Katalysatorbett hat eine Länge von 6,6 m (26 mm-Rohr) bzw. 4,6 m (20 mm-Rohr), wobei es in drei Lagen unterschiedlicher Aktivität geschüttet wird, mit in Flussrichtung steigender Aktivität. Die Länge der unterschiedlich aktiven Zonen, die Katalysatorpartikelgeometrie sowie die Aktivitätsabstufung sind dabei für Fälle mit gleichem Rohrdurchmesser identisch. Stromauf des Katalysatorbetts befindet sich ein Bett aus Inertmaterial (Länge 1,4 m, ebenfalls Ringe gleicher Größe), welches als Vorwärmzone dient.

**Tabelle 1A - Reaktordurchmesser 20 mm, Reaktorlänge 6 m, Gesamthöhe aktiver Schichten 4,6 m, Gesamtlängen-Durchmesser-Verhältnis 230, Aktive Katalysatormasse 0,675 kg**

| | | | | |
|---|---|---|---|---|
| Betriebsdruck [bara] | 3,6 | 4 | 4,5 | 6,1 |
| Kühlmitteltemperatur [°C] | 370 | 380 | 390 | 405 |
| WHSV [kg Einsatz/(h*kg Kat)] | 3,9 | 6,1 | 9,1 | 14,0 |
| Lineargeschwindigkeit [cm/s] | 214,2 | 307,6 | 431,4 | 514,4 |
| Relative molare Ethylenausbeute bez. auf Ethaneinsatz am Reaktoreintritt | 43,2% | 44,1% | 44,8% | 45,3% |
| Ethylenproduktion pro Einzelrohr [kg/h] | 0,69 | 1,09 | 1,66 | 2,58 |
| Benötigte Rohranzahl in Rohrbündelreaktor für 250 kta Ethylen Jahresproduktion | 43345 | 27585 | 18099 | 11625 |
| Kühlfläche pro Einzelrohr [m²] | 0,289 | 0,289 | 0,289 | 0,289 |
| Gesamte Kühlfläche in Rohrbündelreaktor [m²] | 12528 | 7973 | 5231 | 3360 |
| Spezifischer Ethylenertrag pro Kühlfläche [kg/(h*m²)] | 2,39 | 3,76 | 5,74 | 8,93 |
| Spezifische Katalysatorbeladung pro Kühlfläche [kg/m²] | 2,33 | 2,33 | 2,33 | 2,33 |
| Beladung mit Einsatz pro Kühloberfläche [kg/(h*m²)] | 9,19 | 14,14 | 21,20 | 32,69 |

**Tabelle 1B - Reaktordurchmesser 26 mm, Reaktorlänge 8,05 m, Gesamthöhe aktiver Schichten 6,6 m, Gesamtlängen-Durchmesser-Verhältnis 254, Aktive Katalysatormasse 2,07 kg**

| | | | | |
|---|---|---|---|---|
| Betriebsdruck [bara] | 3,5 | 3,5 | 5 | 7 |
| Kühlmitteltemperatur [°C] | 327 | 350 | 355 | 350 |
| WHSV [kg Einsatz/(h*kg Kat)] | 2,3 | 3,2 | 5,6 | 9,0 |
| Lineargeschwindigkeit [cm/s] | 216,2 | 317,2 | 404,4 | 474,0 |
| Relative molare Ethylenausbeute bezogen auf Ethaneinsatz am Reaktoreintritt | 40,6% | 44,0% | 44,5% | 36,9% |
| Ethylenproduktion pro Einzelrohr [kg/h] | 1,15 | 1,71 | 3,06 | 4,09 |
| Benötigte Rohranzahl in Rohrbündelreaktor für 250 kta Ethylen Jahresproduktion | 25997 | 17560 | 9814 | 7344 |
| Kühlfläche pro Einzelrohr [m²] | 0,539 | 0,539 | 0,539 | 0,539 |
| Gesamte Kühlfläche in Rohrbündelreaktor [m²] | 14015 | 9467 | 5291 | 3959 |
| Spezifischer Ethylenertrag pro Kühlfläche [kg/(h*m²)] | 2,14 | 3,17 | 5,67 | 7,58 |
| Spezifische Katalysatorbeladung pro Kühlfläche [kg/m²] | 3,84 | 3,84 | 3,84 | 3,84 |
| Beladung mit Einsatz pro Kühloberfläche [kg/(h*m²)] | 8,89 | 12,17 | 21,53 | 34,64 |

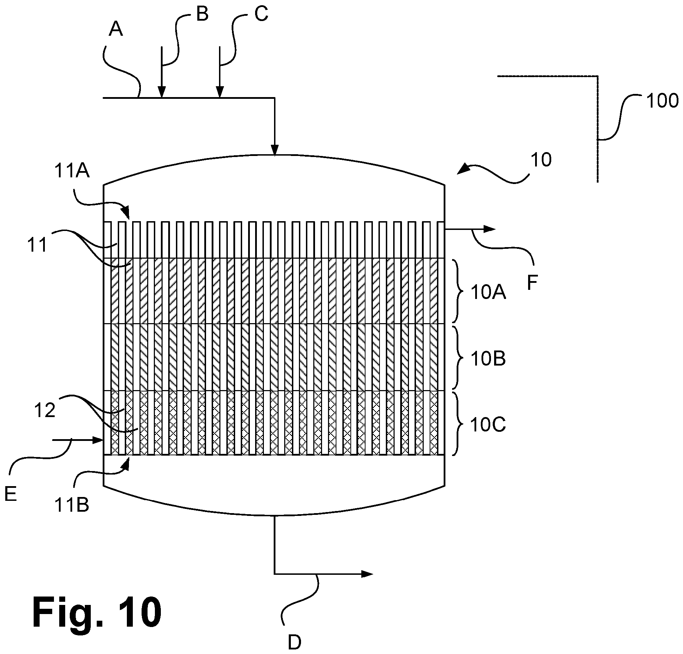

In Figur 10 ist eine Anlage zur Herstellung von Ethylen durch oxidative Dehydrierung von Ethan gemäß einer Ausführungsform der Erfindung in Form eines stark vereinfachten Anlagendiagramms veranschaulicht und insgesamt mit 100 bezeichnet. Die Gesamtanlage ist dabei nur schematisch angedeutet.

Die Anlage 100 weist einen Rohrbündelreaktor 10 mit mehreren und nur zum Teil bezeichneten Reaktionsrohren 11 auf, dem ein Ethan enthaltendes, auf beliebige Weise gewonnenes Gasgemisch in Form eines Stoffstroms A zugeführt wird. Der Stoffstrom A kann beispielsweise einer nicht dargestellten Rektifikationseinheit entnommen werden, die höhere Kohlenwasserstoffe aus einem Ausgangsgemisch abtrennt. Der Stoffstrom A kann auch beispielsweise vorgewärmt und auf andere Weise aufbereitet werden. Der Stoffstrom A kann bereits Sauerstoff und ggf. ein Verdünnungsmittel wie Wasserdampf enthalten; entsprechende Medien können jedoch auch, wie hier stellvertretend in Form von Stoffströmen B und C veranschaulicht, stromauf des Rohrbündelreaktors 10 oder in diesem zugegeben werden.

Die Reaktionsrohre 11 verlaufen durch mehrere, im dargestellten Beispiel drei, Reaktionszonen 10A, 10B, 10C und sind von einem Mantelbereich 12 umgeben. In den Reaktionsrohren 11 sind in den entsprechenden Reaktionszonen 10A, 10B, 10C jeweils Katalysatorbetten vorgesehen und in Form unterschiedlicher Schraffuren veranschaulicht. Ein Ethan sowie Sauerstoff und ggf. ein Verdünnungsmittel enthaltendes Gasgemisch wird Form des Stoffstroms A bzw. der kombinierten Stoffströme A bis C nacheinander durch die Reaktionszonen 10A bis 10C geführt. Den Reaktionszonen 10A bis 10C ist eine inerte Zone vorgeschaltet, die nicht gesondert bezeichnet ist. Die Reaktionsrohre 11 verlaufen zwischen einem ersten Ende 11A und einem zweiten Ende 11B (nur im Fall eines Reaktionsrohrs bezeichnet) und die Reaktionszonen 10A bis 10C sind zwischen diesen Enden 11A, 11B, angeordnet.

Die Reaktionszonen 10A, 10B, 10C zeichnen sich insbesondere durch unterschiedliche Katalysatoraktivitäten in den Katalysatorbetten aus, wie zuvor im Detail erläutert. Die vorliegende Erfindung kann jedoch in anderen Ausgestaltungen auch ohne eine entsprechende Ausbildung unterschiedlicher Reaktionszonen 10A, 10B, 10C bzw. Katalysatorbetten realisiert werden. Alternativ oder zusätzlich kann auch eine zonal unterschiedliche oder einheitliche Temperierung erfolgen.

Aus dem Rohrbündelreaktor 10 strömt ein Prozessgas in Form eines Prozessgasstroms D ab, in dem Ethylen enthalten ist, das in dem Rohrbündelreaktor 10 durch Umsetzung eines Teils des Ethans durch oxidative Dehydrierung gebildet wurde. Ferner enthält das Prozessgas Essigsäure, die ebenfalls in dem Rohrbündelreaktor 10 aus Ethan gebildet wird, sowie Wasser, Kohlenmonoxid, Kohlendioxid, nicht umgesetzten Sauerstoff, sowie das oder die Verdünnungsmittel und weitere Verbindungen, falls zugegeben oder zuvor in dem Rohrbündelreaktor 10 gebildet. Die Reaktionsrohre 11 werden mittels eines durch den Mantelbereich geführten Temperiermittelstroms E temperiert das in Form eines Abflusses F aus dem Mantelbereich ausgeführt wird. Wie hier nicht veranschaulicht, können hierbei insbesondere mehrere Temperiermittelkreisläufe vorgesehen sein, die die Reaktionsrohre 11 abschnittsweise temperieren bzw. kühlen. Eine Wandung der Reaktionsrohre 11 im Bereich der jeweiligen Katalysatorbetten definiert dabei eine jeweils eine aktive Kühlfläche für die Katalysatorbetten.

Es versteht sich, dass die Anlage 100 wie veranschaulicht einen, aber auch mehrere, beispielsweise parallel betriebene, Rohrbündelreaktoren 10 aufweisen kann. In letzterem Fall werden diesen Rohrbündelreaktoren 10 jeweils entsprechende Reaktionseinsätze, die gleich oder unterschiedlich zusammengesetzt sein können, zugeführt, und es werden jeweils entsprechende Prozessgasströme D gebildet. Letztere können vereinigt und gemeinsam als Prozessgas nachfolgenden Verfahrensschritten bzw. Anlagenteilen zugeführt werden.

Stromab des Rohrbündelreaktors 10 kann ein Wasserpartialdruck erfasst werden. Dieser kann beispielsweise durch eine Zugabe von Wasser bzw. Dampf zu dem Gasgemisch des Stoffstroms A bzw. in Form der Stoffströme B oder C eingestellt werden. Eine weitere Beeinflussung, insbesondere eine Feineinstellung, kann durch Einstellen der Temperatur in dem Rohrbündelreaktor 10 erfolgen.

Nachfolgende Verfahrensschritte bzw. Anlagenkomponenten sind nicht veranschaulicht. Das Prozessgas kann in diesen mit Waschwasser oder einer geeigneten wässrigen Lösung in Kontakt gebracht werden, wodurch das Prozessgas insbesondere abgekühlt und Essigsäure aus dem Prozessgas ausgewaschen werden kann. Das zumindest weitgehend von Essigsäure befreite Prozessgas kann weiter aufbereitet und einer Abtrennung von Ethylen unterworfen werden. In dem Prozessgas enthaltendes Ethan kann in den Reaktor 10 zurückgeführt werden.

## Patentansprüche

**1.** Verfahren zur Herstellung von Ethylen durch oxidative Dehydrierung von Ethan, bei dem ein Rohrbündelreaktor (10) mit Reaktionsrohren (11) verwendet wird, die sich zwischen einem ersten Ende (11A) und einem zweiten Ende (11B) erstrecken, wobei in jedem der Reaktionsrohre (11) ein oder mehrere Katalysatorbetten angeordnet sind und in jedem der Reaktionsrohre (11) ein Verhältnis einer Gesamtlänge des einen oder der mehreren Katalysatorbetten zwischen dem ersten Ende (11A) und dem zweiten Ende (11B) zu einem Durchmesser jedes der Reaktionsrohre (11) einen Wert zwischen 150 und 400 aufweist, **dadurch gekennzeichnet, dass** der Rohrbündelreaktor (10) mit einer Lineargeschwindigkeit von 250 bis 800 cm/s betrieben wird, und dass das eine oder die mehreren Katalysatorbetten derart ausgestaltet werden, dass ein Verhältnis von aktiver Katalysatormasse zu wirksamer Kühlfläche in einem Bereich zwischen 1,5 und 5 kg/m² liegt.

**3.** Verfahren nach Anspruch 1, bei dem der Rohrbündelreaktor (10) mit einer gewichtsbezogenen stündlichen Raumgeschwindigkeit von mehr als 3 oder 5 kg und weniger als 20 kg Einsatz pro Stunde und kg Katalysator betrieben wird.

**4.** Verfahren nach Anspruch 1, bei dem der Rohrbündelreaktor (10) mit einer Lineargeschwindigkeit von mehr als 300, 400 oder 500 cm/s betrieben wird.

**5.** Verfahren nach einem der vorstehenden Ansprüche, bei dem der Rohrbündelreaktor (10) so betrieben wird, dass eine Maximaltemperatur in dem einen oder den mehreren Katalysatorbetten weniger als 500 oder 450 °C beträgt.

**6.** Verfahren nach einem der vorstehenden Ansprüche, bei dem in dem einen oder den mehreren Katalysatorbetten Füllkörper verwendet werden, die aus Ringen, Pellets und Extrudaten oder Kombinationen hiervon ausgewählt sind.

**7.** Verfahren nach einem der vorstehenden Ansprüche, bei dem mehrere Katalysatorbetten mit unterschiedlicher Katalysatoraktivität verwendet werden.

**8.** Verfahren nach einem der vorstehenden Ansprüche, bei dem in Abhängigkeit von einer detektierten Katalysatoraktivität in dem Rohrbündelreaktor (10) eine Wassereinspeisung in den Rohrbündelreaktor (10) dosiert wird.

**9.** Verfahren nach einem der vorstehenden Ansprüche, bei dem der Rohrbündelreaktor (10) mit einem oder mehreren Kühlmedien durchströmt wird.

**10.** Verfahren nach Anspruch 9, bei dem unterschiedliche Bereiche des Rohrbündelreaktors (10) in unterschiedlichem Ausmaß gekühlt werden.

**11.** Anlage (100) zur Herstellung von Ethylen durch oxidative Dehydrierung von Ethan, die einen Rohrbündelreaktor (10) mit Reaktionsrohren (11) aufweist, die sich zwischen einem ersten Ende (11A) und einem zweiten Ende (11B) erstrecken, wobei in jedem der Reaktionsrohre (11) ein oder mehrere Katalysatorbetten angeordnet sind und in jedem der Reaktionsrohre (11) ein Verhältnis einer Gesamtlänge des einen oder der mehreren Katalysatorbetten zwischen dem ersten Ende (11A) und dem zweiten Ende (11B) zu einem Durchmesser jedes der Reaktionsrohre (11) einen Wert zwischen 150 und 400 aufweist, **dadurch gekennzeichnet, dass** der Rohrbündelreaktor (10) für einen Betrieb mit einer Lineargeschwindigkeit von 250 bis 800 cm/s eingerichtet ist und das eine oder die mehreren Katalysatorbetten derart ausgestaltet sind, dass ein Verhältnis von aktiver Katalysatormasse zu wirksamer Kühlfläche in einem Bereich zwischen 1,5 und 5 kg/m² liegt.

**12.** Anlage nach Anspruch 11, die zur Durchführung eines Verfahrens nach einem der Ansprüche 1 bis 9 eingerichtet ist.
